(19) Europäisches Patentamt
European Patent Office
Office européen des brevets

(11) EP 3 773 930 B1

(12) # FASCICULE DE BREVET EUROPEEN

(45) Date de publication et mention de la délivrance du brevet:
**11.03.2026 Bulletin 2026/11**

(21) Numéro de dépôt: **19723154.1**

(22) Date de dépôt: **12.04.2019**

(51) Classification Internationale des Brevets (IPC):
***A61Q 19/00*** *(2006.01)* ***A61Q 19/10*** *(2006.01)*
***A61K 8/368*** *(2006.01)* ***A61P 17/10*** *(2006.01)*

(52) Classification Coopérative des Brevets (CPC):
**A61Q 19/008; A61K 8/368; A61P 17/10; A61Q 19/10**

(86) Numéro de dépôt international:
**PCT/FR2019/050869**

(87) Numéro de publication internationale:
**WO 2019/197788 (17.10.2019 Gazette 2019/42)**

(54) **UTILISATION D'UNE NOUVELLE COMPOSITION POUR EMPÊCHER OU RALENTIR L'APPARITION DES SIGNES INESTHÉTIQUES LIÉS À LA PRÉSENCE DE SÉBUM EN EXCÈS**

VERWENDUNG EINER NEUEN ZUSAMMENSETZUNG ZUR VERHINDERUNG ODER VERLANGSAMUNG DES AUFTRETENS VON UNSCHÖNEN ANZEICHEN IN BEZUG AUF DAS VORHANDENSEIN VON ÜBERSCHÜSSIGEM TALG

USE OF A NOVEL COMPOSITION FOR PREVENTING OR SLOWING DOWN THE APPEARANCE OF UNSIGHTLY SIGNS RELATING TO THE PRESENCE OF EXCESS SEBUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.04.2018 FR 1853257**

(43) Date de publication de la demande:
**17.02.2021 Bulletin 2021/07**

(73) Titulaire: **Société d'Exploitation de Produits pour les Industries Chimiques SEPPIC**
**75321 Paris cedex 07 (FR)**

(72) Inventeurs:
- **KERN, Catherine**
**75321 PARIS CEDEX 07 (FR)**
- **GARCIA, Christine**
**81100 CASTRES (FR)**
- **GOMBERT, Christian**
**75321 PARIS CEDEX 07 (FR)**
- **MSIKA, Philippe**
**PARIS CEDEX 07 PARIS (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**WO-A2-2006/127525** **CN-A- 107 595 713**
**FR-A1- 2 877 572** **FR-A1- 2 877 572**
**US-A- 4 363 815** **US-A- 4 529 587**
**US-A1- 2017 239 159**

- **JIANG XIAO-WEN ET AL: "Caffeoylquinic acid derivatives from the roots ofArctium lappaL. (burdock) and their structure-activity relationships (SARs) of free radical scavenging activities", PHYTOCHEMISTRY LETTERS, vol. 15, 8 January 2016 (2016-01-08), pages 159 - 163, XP029459897, ISSN: 1874-3900, DOI: 10.1016/J.PHYTOL.2015.12.008**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- XIAOWEI ZOU ET AL: "Isolation and characterization of two new phenolic acids from cultured cells of Saussurea involucrata", PHYTOCHEMISTRY LETTERS, vol. 7, 1 February 2014 (2014-02-01), AMSTERDAM, NL, pages 133 - 136, XP055523570, ISSN: 1874-3900, DOI: 10.1016/j.phytol.2013.11.002

## Description

La présente invention a pour objet l'utilisation d'une composition comprenant des dérivés d'acides quiniques poly-substitués, et plus particulièrement d'un extrait de la plante *Arctium lappa* comprenant lesdits dérivés pour préparer des formulations à usage topique destinées à diminuer la quantité de sébum produite par la peau humaine et/ou le cuir chevelu.

La peau humaine constituant la première image offerte au regard d'autrui, l'amélioration de son aspect est souvent un sujet de préoccupation pour l'être humain. La peau est le reflet soit d'un état de bien-être, souvent associé à une peau nette/éclatante, soit, a contrario, à un état de fatigue et/ou de négligence, souvent associé à une peau grasse ou reluisante.

La peau est un organe atypique du corps humain, extrêmement fin au regard de son étendue mais également l'organe le plus lourd d'un individu. Une des caractéristiques de la peau réside dans le fait qu'il s'agit d'un organe d'interface, d'un organe limite, entre le milieu intérieur (corps humain) et le milieu extérieur. De ce fait, et avec la flore qui la recouvre et l'habite, la peau est la première barrière de protection de l'organisme humain.

En raison de sa position d'interface avec le milieu extérieur, la peau est soumise à des sollicitations quotidiennes nombreuses, telles que par exemple le contact avec des vêtements, les changements de températures, les changements de degrés d'hygrométrie, les changements de pression, voire à des agressions, comme par exemple le contact avec certains produits chimiques présentant ou pouvant présenter un caractère très acide, ou très basique, ou irritant, avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui. Par conséquent, l'amélioration de son aspect est un sujet de préoccupation constant pour les êtres humains. La peau est le reflet d'un état de bien-être, souvent associé à la jeunesse, et *a contrario* d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.

A la périphérie de l'épiderme, se trouve une couche cornée supérieure, nommée le *stratum corneum,* qui est la première couche de l'épiderme à subir les stress d'origine externe, comme les variations de conditions climatiques extérieures (température, pression, hygrométrie) ou les sollicitations mécaniques.

Le *stratum corneum* est plus particulièrement en contact avec le microbiote cutané.

Par « microbiote cutané », on désigne au sens de la présente demande une population de micro-organismes, spécialisés ou opportunistes, comme par exemple les bactéries, les champignons, les levures..., qui vit à la surface de la peau.

Le microbiote cutané ne peut pas être défini de façon spécifique et généralisé pour l'ensemble des individus. Depuis le lancement en 2007 du projet « Human Microbiome Project » (HMP) du National Institute of Health, les chercheurs ont pu observer de grandes variations topographiques du microbiote humain ainsi que de grandes différences selon les individus.

Au moins dix-neuf *phyla* ont été identifiés dont les quatre principaux sont Actinobacteria (51,8%), Firmicutes (24,4%), Proteobacteria (16,5%) et Bacteroidetes (6,3%). Les genres majoritairement identifiés sont *Corynebacterium, Propionibacterium* et *Staphylococcus.* L'abondance de chaque groupe est fortement dépendante des différentes localisations. Les organismes fongiques isolés sur la peau sont du genre *Malassezia* spp. Par ailleurs, des acariens du genre Demodex sont également présents et résident dans les unités pilo-sébacées, le plus souvent de la surface du visage.

Ce microbiote s'alimente à la fois de molécules excrétées par la peau (lipides, protéines...), et de composés

sécrétés par les communautés de micro-organismes mettant en évidence une réelle coopération au sein de ce microbiote. De plus, cette relation avec l'hôte constitue une véritable symbiose.

Les bactéries peuvent être commensales lorsqu'elles vivent au contact du revêtement cutanéo-muqueux d'un hôte sans entraîner de dommages. Un équilibre s'installe alors entre l'individu et les flores diverses commensales de la peau et des muqueuses, mais cet équilibre est sans cesse menacé par les agressions physiques ou chimiques subies par le *stratum corneum,* comme par exemple la pollution, les variations de température, les rayonnements ultra-violet, l'utilisation intensive de produits tensioactifs détergents, le stress, etc.... A côté de ces bactéries commensales, se trouvent les bactéries transitoires, indésirables et/ou pathogènes.

*Staphylococcus epidermidis (S. epidermidis)* constitue plus de 90% de la flore résidente en aérobie présente dans le *stratum corneum.* La flore résidente est aussi composée de bactéries anaérobie appartenant à la division des Actinobactéries comme *Propionibacterium acnes* (P. *acnes),* fréquemment retrouvés au niveau des zones sébacées, comme par exemple le dos, le visage et le cuir chevelu.

Alors que la flore normale de la peau constitue une défense pour l'hôte, une augmentation ou une réduction de la composition bactérienne (dysbiose) peut conduire à l'inflammation cutanée et peut être à l'origine du développement de certaines pathologies cutanées comme l'acné, la dermatite atopique ou même le psoriasis.

La peau grasse est liée à un phénomène biologique appelé hyperséborrhée ou hyper-sécrétion sébacée, définie comme étant une production anormalement élevée de sébum par la glande sébacée de la peau. Ce sébum, s'écoulant à la surface de la peau, lui confère ainsi cette caractéristique disgracieuse « luisante ». Cette hyperséborrhée est généralement liée à une présence en excès de l'hormone testostérone qui, une fois dans la glande sébacée, est transformée par la 5-alpha réductase en dihydrotestostérone (DHT), qui se lie aux récepteurs présents dans les cellules sébacées et active la synthèse de sébum.

L'hyperséborrhée s'accompagne d'une hyperkératinisation folliculaire, caractérisée par une augmentation du nombre de kératinocytes au niveau de l'infundibulum folliculaire, qui provoque une obstruction du canal folliculaire et rend difficile l'écoulement du sébum.

Ceci entraîne une accumulation du sébum qui est profitable au développement de la bactérie anaérobie *Propionibacterium acnes.* Cette dernière va entraîner l'apparition d'un phénomène inflammatoire. L'ensemble de ces phénomènes conduit à l'apparition de signes cliniques caractéristiques, tels que des comédons, caractérisant les peaux acnéiques.

La présence de la bactérie P. *acnes* au niveau de la structure pilo-sébacée est connue pour être impliquée dans le développement de la pathologie. L'acné vulgaire est une maladie de la peau qui est liée à une dysbiose, i.e. un déséquilibre du microbiote cutané. Une consommation excessive de tabac et d'alcool semble également appartenir aux facteurs susceptibles d'accentuer les effets de l'acné vulgaris. Parmi les autres facteurs environnementaux le stress, la pollution urbaine ainsi que le soleil (rayonnement UV induisant la peroxydation du sébum et en particulier du squalène), sont également reconnus comme étant impliqués dans l'aggravation des symptômes de l'acné vulgaris.

La colonisation du follicule pilo-sébacé par P. *acnes* est un facteur important pour la réaction inflammatoire dans l'acné vulgaire. De fait, l'acné n'est pas *sensu stricto* une maladie infectieuse car ce germe exerce d'abord une action inflammatoire, liée à ses très nombreuses sécrétions enzymatiques et chimiques et aux réactions immunologiques qu'il provoque. Ainsi, P. *acnes* stimule la production par les sébocytes, les kératinocytes et les leucocytes (lymphocytes et monocytes) de nombreuses cytokines inflammatoires (IL-1$\alpha$, IL1$\beta$, IL-6, IL-8, IL-10, IL-12, IL-17, IL-18, TNF-$\alpha$, GM-CSF et IFN-y) ainsi que des peptides antimicrobiens (défensines et cathélicidines), des métalloprotéinases matricielles, des espèces réactives de l'oxygène et d'autres produits impliqués dans la réaction inflammatoire.

De plus, P. *acnes* sécrète une lipase qui hydrolyse les triglycérides du sébum en acides gras libres qui sont irritants et chimiotactiques pour les neutrophiles.

Parmi les extraits de végétaux que l'on peut utiliser pour leurs actions sur le microbiote humain, on peut citer un extrait lyophilisé de feuilles de bardane (ou *Arctium lappa)* pour lequel une activité antibactérienne a été mise en évidence et plus particulièrement une activité contre des micro-organismes oraux, se montrant plus efficaces contre des bactéries associées à des pathogènes endodontiques tels que *Bacillus subtilis, Candida albicans, Lactobacillus acidophilus* et *Pseudomonas aeruginosa (1).*

Il est également décrit que les feuilles de bardane sont aussi un remède topique possible pour des problèmes de peau tels que l'eczéma, l'acné et le psoriasis (1).

Il est également décrit dans la littérature que les extraits de feuilles de bardane montrent des activités antimicrobiennes (2).

La demande de brevet chinois publiée sous le numéro CN106074663 A décrit une composition d'extraits de plantes comprenant un extrait de bois de Milo, un extrait de racines de bardane, et un extrait de chèvrefeuille, et décrit plus particulièrement que l'extrait de racines de Bardane traite les peaux sèches, le prurit aigu, l'inflammation, les cicatrices et d'autres symptômes, en inhibant des facteurs inflammatoires induits par différentes causes (stress externe ou facteurs génétiques), améliorant l'activité immunitaire et l'activité anti-oxydante de la peau, apaisant et réparant la peau.

La demande de brevet américain publiée sous le numéro US20170136077 divulgue qu'un certain nombre

d'extraits de végétaux, parmi lesquels un extrait racinaire de bardane, un extrait racinaire *d'Epilobium angustifolium,* un extrait de *Cystoseira amentacea* favorisent la réduction de la production des cytokines IL-8, IL-1 et TNF-$\alpha$ par des kératinocytes stimulés par du Phorbol 12-myristate 13-acétate (ou « PMA »).

Ce modèle est connu pour évaluer l'effet anti-inflammatoire d'ingrédients car le PMA est un activateur de la voie protéine kinase C qui a un effet inflammatoire général sur les cellules.

La demande de brevet américain publiée sous le numéro US2017/239159 A1 décrit une composition capable de contrôler la production de sébum en régulant l'expression d'antigènes ABH comprenant l'acide 1,3-dicaffeoylquinique, l'acide 1,5-dicaffeoylquinique, l'amentoflavone ou un dérivé de ceux-ci ou un sel pharmaceutiquement acceptable de ceux-ci.

Dans le cadre de leurs recherches concernant de nouveaux actifs cosmétiques pour la prévention et/ou le traitement des signes des effets inesthétiques liés à la présence d'une quantité en excès de sébum sur la peau et/ou le cuir chevelu, comme par exemple une teneur en sébum sur la peau supérieure à 95 $\mu$grammes.cm$^{-2}$ ou plus particulièrement supérieure à 120 $\mu$grammes.cm$^{-2}$, les inventeurs se sont attachés à développer une nouvelle solution technique fondée sur l'utilisation d'une composition comprenant des acides quiniques poly-substitués (ou « QPS »), sur l'utilisation d'extraits de racines de Bardane comprenant lesdites QPS, obtenus par un procédé comprenant une étape préalable de culture en aéroponie de ladite Bardane, pour présenter des effets de diminution de sébum sur la peau humaine.

Selon un premier aspect, l'invention a pour objet un procédé de préparation d'une composition ($C_1$) comprenant pour 100% de sa masse :

**a)** - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;

**b)** - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

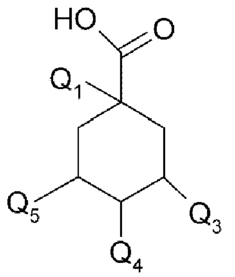

(I),

dans laquelle $Q_1$, $Q_3$, $Q_4$ et $Q_5$ représentent indépendamment les uns des autres, le radical hydroxyle ou un ses sels ou un radical choisi parmi :

**(i)** - Le radical caféoyle de formule (II) :

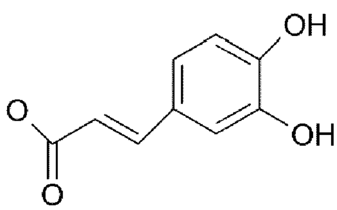

(II)

**(ii)** - Le radical maloyle de formule (IIIa) ou (IIIb) :

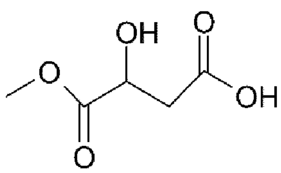

(IIIa)

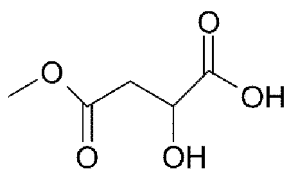

(IIIb) ;

**(iii)** - Le radical caféoyl maloyle de formule (IVa) ou (IVb) :

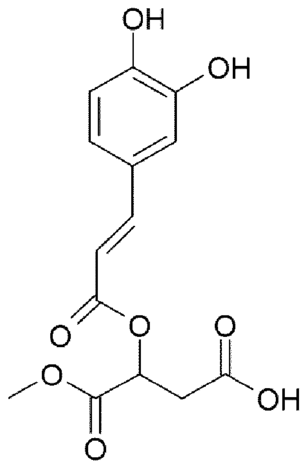

(IVa)

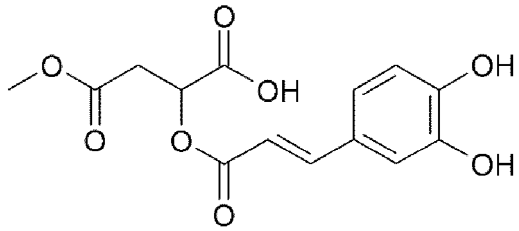

(IVb)

**(iv)** - Le radical maloyl caféoyle de formule (Va), (Vb), (Vc) ou (Vd),

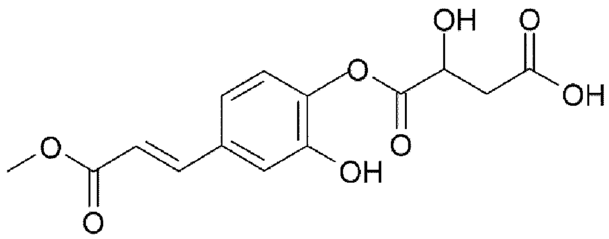

(Va)

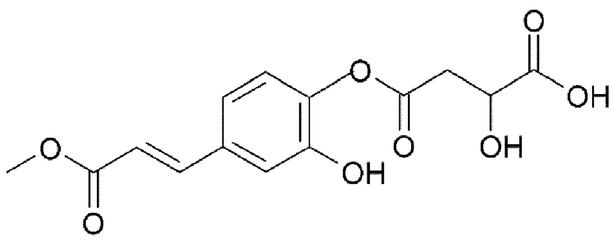

(Vb)

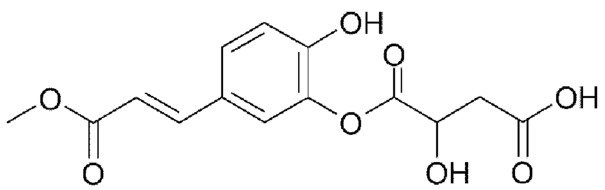

(Vc),

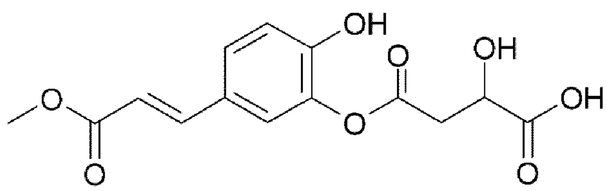

(Vd)

étant entendu qu'au moins un de ces radicaux $Q_1$, $Q_3$, $Q_4$ et $Q_5$ ne représente ni le radical - OH ; ni un de ses sels ; et

- c) - De 20,0% massique à 35,0% massique d'eau,

pour empêcher ou ralentir l'apparition des signes inesthétiques liés à la présence de sébum en excès sur la peau et/ou le cuir chevelu chez l'être humain ; ledit procédé comprenant les étapes suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/milieu ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;
- Une étape c) de séparation des racines de la biomasse à l'issue du traitement défini à l'étape b), pour isoler une phase liquide ($L_1$) ;
- Une étape d) d'immersion de ladite biomasse ($BM_2$) issue de l'étape c) dans ledit milieu ($S_1$); dans un ratio biomasse ($BM_2$)/milieu ($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$) .
- Une étape f) de filtration de ladite phase liquide ($L_2$) obtenue à l'étape e), pour isoler une phase liquide liquide ($L_3$),
- Une étape g) de mélange des dites phases liquides ($L_1$) et ($L_3$), puis si nécessaire d'addition d'eau et/ou dudit solvant organique ($SO_1$), de façon à obtenir la composition ($C_1$).

Au sens de la présente invention, par « signes inesthétiques liés à la présence de sébum sur la peau et/ou le cuir chevelu », on entend au sens de l'invention, toutes modifications de l'aspect extérieur de la peau ou du cuir chevelu dues à une quantité en excès de sébum sur ladite peau ou ledit cuir chevelu, comme par exemple un aspect luisant, un aspect gras, une sensation collante de la peau ou du cuir chevelu, la présence de comédons fermés (points blancs) et les comédons ouverts (points noirs) sur la peau.

Au sens de la présente invention, par « excès de sébum sur la peau ou le cuir chevelu », on entend une teneur en sébum sur la peau supérieure à 95 $\mu$grammes.cm$^{-2}$ ou plus particulièrement supérieure à 120 $\mu$grammes.cm$^{-2}$.

Au sens de la présente invention, l'expression «ladite quantité massique $x_1$ étant exprimée en équivalent massique de l'acide 1-O-(2-caféoyl)maloyl-3,5-O-dicaféoyl quinique » signifie que la quantité massique $x_1$ a été déterminée par la mise en œuvre d'une méthode analytique quantitative de type UHPLC-MS (« Ultra High Performance Liquid Chromatography-Mass Spectra), utilisant comme étalon de référence un standard de l'acide 1-O-(2-caféoyl) maloyl-3,5-O-dicaféoyl quinique préalablement isolé et purifié à une teneur supérieure ou égale à 99%.

Une telle analyse quantitative de type UHPLC-MS a été réalisée avec un appareillage de type UHPLC-MS Shimadzu_Nexera_LCMS 2020, équipé d'un détecteur à barrette de diode et réglé à une longueur d'onde de 330 nanomètres, d'une colonne de type Kinetex 2,6u XB-C18 100A. 100 x 2,1, et mettant en œuvre une phase mobile A composée d'eau et de 0,1% massique d'acide formique et une phase mobile B constituée par l'acétonitrile,

Parmi les composés de formule générale (I) présents dans la composition (ES), on peut citer :

- les composés de la famille des acides DiCaféoylQuiniques (DCQ) tels que décrit dans le tableau 1 ci-dessous :

**Tableau 1**

| **Acide DiCaféoylQuiniques (DCQ)** | | | |
|---|---|---|---|
| **Acide 1,3-O-dicaféoylquinique (1,3-DCQ)** | | | |
| **Q1** | **Q3** | **Q4** | **Q5** |
| Caféoyl | Caféoyl | OH | OH |
| **Acide 1,4-O-dicaféoylquinique (1,4-DCQ)** | | | |
| **Q1** | **Q3** | **Q4** | **Q5** |
| Caféoyl | OH | Caféoyl | OH |

(suite)

| Acide 1,5-O-dicaféoylquinique (1,5-DCQ) | | | |
|---|---|---|---|
| **Q1** | **Q3** | **Q4** | **Q5** |
| Caféoyl | OH | OH | Caféoyl |
| **Acide 3,4-O-dicaféoylquinique (3,4-DCQ)** | | | |
| **Q1** | **Q3** | **Q4** | **Q5** |
| OH | Caféoyl | Caféoyl | OH |
| **Acide 3,5-O-dicaféoylquinique (3,5-DCQ)** | | | |
| **Q1** | **Q3** | **Q4** | **Q5** |
| OH | Caféoyl | OH | Caféoyl |
| **Acide 4,5-O-dicaféoylquinique (4,5 DCQ)** | | | |
| **Q1** | **Q3** | **Q4** | **Q5** |
| OH | OH | Caféoyl | Caféoyl |

- les composés de la famille des acides TriCaféoylQuiniques (TCQ) tels que décrit dans le tableau 2 ci-dessous :

**Tableau 2**

| Acide TriCaféoylQuinique (TCQ) | | | |
|---|---|---|---|
| **Acide 1,3,4-O-tricaféoylquinique (1,3,4-TCQ)** | | | |
| **Q1** | **Q3** | **Q4** | **Q5** |
| Caféoyl | Caféoyl | Caféoyl | OH |
| **Acide 1,3,5-O-tricaféoylquinique (1,3,5-TCQ)** | | | |
| **Q1** | **Q3** | **Q4** | **Q5** |
| Caféoyl | Caféoyl | OH | Caféoyl |
| **Acide 1,4,5-O-tricaféoylquinique (1,4,5-TCQ)** | | | |
| **Q1** | **Q3** | **Q4** | **Q5** |
| Caféoyl | OH | Caféoyl | Caféoyl |
| **Acide 3,4,5-O-tricaféoylquinique (1,3,4-TCQ)** | | | |
| **Q1** | **Q3** | **Q4** | **Q5** |
| OH | Caféoyl | Caféoyl | Caféoyl |

- les composés de la famille des acides Maloyl TriCaféoylQuiniques (m-TCQ) tels que décrit dans le tableau 3 ci-dessous :

**Tableau 3**

| Maloyl TriCaféoylQuiniques (m-TCQ) | | | |
|---|---|---|---|
| **Acide 1-O-maloyl-(3,4,5-O-tricaféoyl)quinique** | | | |
| **Q1** | **Q3** | **Q4** | **Q5** |
| Maloyl | Caféoyl | Caféoyl | Caféoyl |
| **Acide 3-O-maloyl-(1,4,5-O-tricaféoyl)quinique** | | | |
| **Q1** | **Q3** | **Q4** | **Q5** |
| Caféoyl | Maloyl | Caféoyl | Caféoyl |

(suite)

| Acide 4-O-maloyl-(1,3,5-O-tricaféoyl)quinique | | | |
|---|---|---|---|
| **Q1** | **Q3** | **Q4** | **Q5** |
| Caféoyl | Caféoyl | Maloyl | Caféoyl |
| **Acide 5-O-maloyl-(1,3,4-O-tricaféoyl)quinique** | | | |
| **Q1** | **Q3** | **Q4** | **Q5** |
| Caféoyl | Caféoyl | Caféoyl | Maloyl |

- les composés de la famille des acides Maloyl DiCaféoylQuiniques (m-DCQ) tels que décrit dans le tableau 4 ci-dessous :

**Tableau 4**

| Acide Maloyl DiCaféoylQuinique (m-DCQ) | | | |
|---|---|---|---|
| **Acide 4-O-maloyl- (1,3-O-dicaféoyl)quinique** | | | |
| **Q1** | **Q3** | **Q4** | **Q5** |
| Caféoyl | Caféoyl | Maloyl | OH |
| Acide 5-O-maloyl- (1,3-O-dicaféoyl)quinique | | | |
| Q1 | Q3 | Q4 | Q5 |
| Caféoyl | Caféoyl | OH | Maloyl |
| **Acide 3-O-maloyl-** (1,4-O-dicaféoyl)quinique | | | |
| Q1 | Q3 | Q4 | Q5 |
| Caféoyl | Maloyl | Caféoyl | OH |
| **Acide 5-O-maloyl- (1,4-O-dicaféoyl)quinique** | | | |
| **Q1** | **Q3** | **Q4** | **Q5** |
| Caféoyl | OH | Caféoyl | Maloyl |
| Acide 3-O-maloyl- (1,5-O-dicaféoyl)quinique | | | |
| Q1 | Q3 | Q4 | Q5 |
| Caféoyl | Maloyl | OH | Caféoyl |
| **Acide 4-O-maloyl- (1,5-O-dicaféoyl)quinique** | | | |
| **Q1** | **Q3** | **Q4** | **Q5** |
| Caféoyl | OH | Maloyl | Caféoyl |
| **Acide 1-O-maloyl- (3,4-O-dicaféoyl)quinique** | | | |
| **Q1** | **Q3** | **Q4** | **Q5** |
| Maloyl | Caféoyl | Caféoyl | OH |
| **Acide 5-O-maloyl- (3,4-O-dicaféoyl)quinique** | | | |
| **Q1** | **Q3** | **Q4** | **Q5** |
| OH | Caféoyl | Caféoyl | Maloyl |
| **Acide 1-O-maloyl- (3,5-O-dicaféoyl)quinique** | | | |
| **Q1** | **Q3** | **Q4** | **Q5** |
| Maloyl | Caféoyl | OH | Caféoyl |

(suite)

| Acide 4-O-maloyl- (3,5-O-dicaféoyl)quinique | | | |
|---|---|---|---|
| **Q1** | **Q3** | **Q4** | **Q5** |
| OH | Caféoyl | Maloyl | Caféoyl |
| **Acide 1-O-maloyl- (4,5-O-dicaféoyl)quinique** | | | |
| **Q1** | **Q3** | **Q4** | **Q5** |
| Maloyl | OH | Caféoyl | Caféoyl |
| **Acide 3-O-maloyl- (4,5-O-dicaféoyl)quinique** | | | |
| **Q1** | **Q3** | **Q4** | **Q5** |
| OH | Maloyl | Caféoyl | Caféoyl |

- les composés de la famille des acides CaféoylMaloyl TriCaféoylQuiniques tels que décrit dans le tableau 5 ci-dessous :

**Tableau 5**

| Acide CaféoylMaloyl TriCaféoylQuinique (cm-TCQ) | | | |
|---|---|---|---|
| **Acide 1-O--(2-caféoyl)maloyl -(3,4,5-O-tricaféoyl)quinique** | | | |
| **Q1** | **Q3** | **Q4** | **Q5** |
| CaféoylMaloyl | Caféoyl | Caféoyl | Caféoyl |
| **Acide 3-O--(2-caféoyl)maloyl-(1,4,5-O-tricaféoyl)quinique** | | | |
| **Q1** | **Q3** | **Q4** | **Q5** |
| Caféoyl | CaféoylMaloyl | Caféoyl | Caféoyl |
| **Acide 4-O--(2-caféoyl)maloyl-(1,3,5-O-tricaféoyl)quinique** | | | |
| **Q1** | **Q3** | **Q4** | **Q5** |
| Caféoyl | Caféoyl | CaféoylMaloyl | Caféoyl |
| **Acide 5-O-(2-caféoyl)maloyl-(1,3,4-O-tricaféoyl)quinique** | | | |
| **Q1** | **Q3** | **Q4** | **Q5** |
| Caféoyl | Caféoyl | Caféoyl | CaféoylMaloyl |

- les composés de la famille des acides CaféoylMaloyl DiCaféoylQuiniques tels que décrit dans le tableau 6 ci-dessous

**Tableau 6**

| Acide CaféoylMaloyl DiCaféoylQuinique (cm-DCQ) | | | |
|---|---|---|---|
| **Acide 4-O-(2-caféoyl)maloyl (1,3-O-dicaféoyl)quinique** | | | |
| **Q1** | **Q3** | **Q4** | **Q5** |
| Caféoyl | Caféoyl | CaféoylMaloyl | OH |
| Acide 5-O-(2-caféoyl)maloyl (1,3-O-dicaféoyl)quinique | | | |
| **Q1** | **Q3** | **Q4** | **Q5** |
| Caféoyl | Caféoyl | OH | CaféoylMaloyl |
| **Acide 3-O-(2-caféoyl)maloyl- (1,4-O-dicaféoyl)quinique** | | | |
| **Q1** | **Q3** | **Q4** | **Q5** |
| Caféoyl | CaféoylMaloyl | Caféoyl | OH |

(suite)

| Acide 5-O-(2-caféoyl)maloyl -(1,4-O-dicaféoyl)quinique | | | |
|---|---|---|---|
| **Q1** | **Q3** | **Q4** | **Q5** |
| Caféoyl | OH | Caféoyl | CaféoylMaloyl |
| **Acide 3-O-(2-caféoyl)maloyl - (1,5-O-dicaféoyl)quinique** | | | |
| **Q1** | **Q3** | **Q4** | **Q5** |
| Caféoyl | CaféoylMaloyl | OH | Caféoyl |
| **Acide 4-O-(2-caféoyl)maloyl - (1,5-O-dicaféoyl)quinique** | | | |
| **Q1** | **Q3** | **Q4** | **Q5** |
| Caféoyl | OH | CaféoylMaloyl | Caféoyl |
| **Acide 1-O-(2-caféoyl)maloyl - (3,4-O-dicaféoyl)quinique** | | | |
| **Q1** | **Q3** | **Q4** | **Q5** |
| CaféoylMaloyl | Caféoyl | Caféoyl | OH |
| Acide 5-O-(2-caféoyl)maloyl - (3,4-O-dicaféoyl)quinique | | | |
| Q1 | Q3 | Q4 | Q5 |
| OH | Caféoyl | Caféoyl | CaféoylMaloyl |
| **Acide 1-O-(2-caféoyl)maloyl - (3,5-O-dicaféoyl)quinique** | | | |
| **Q1** | **Q3** | **Q4** | **Q5** |
| Maloyl | Caféoyl | OH | Caféoyl |
| **Acide 4-O-(2-caféoyl)maloyl - (3,5-O-dicaféoyl)quinique** | | | |
| **Q1** | **Q3** | **Q4** | **Q5** |
| OH | Caféoyl | CaféoylMaloyl | Caféoyl |
| **Acide 1-O-(2-caféoyl)maloyl - (4,5-O-dicaféoyl)quinique** | | | |
| **Q1** | **Q3** | **Q4** | **Q5** |
| CaféoylMaloyl | OH | Caféoyl | Caféoyl |
| **Acide 3-O-(2-caféoyl)maloyl - (4,5-O-dicaféoyl)quinique** | | | |
| **Q1** | **Q3** | **Q4** | **Q5** |
| OH | CaféoylMaloyl | Caféoyl | Caféoyl |

Selon un aspect particulier de la présente invention, la composition (ES) telle que définie précédemment comprend au moins :

- Au moins un composé de formule (Ia) correspondant à la formule (I) pour laquelle $Q_1$ représente le radical maloyle de formule (IIIa) ou de formule (IIIb) et $Q_3$ et $Q_4$ et $Q_5$ identiques, représentent chacun le radical caféoyle de formule (II) ;
- Un composé de formule (Ib) correspondant à la formule (I) pour laquelle $Q_1$ représente le radical caféoylmaloyle de formule (IVa) ou de formule (IVb), $Q_3$ et $Q_5$ représentent chacun le radical caféoyle de formule (II) et $Q_4$ représente le radical hydroxyle, et
- Au moins un composé de formule (Ic) choisi parmi :

  - Le composé de formule ($Ic_1$) correspondant à la formule (I) pour laquelle $Q_1$ et $Q_5$ représentent chacun le radical caféoyle de formule (II), $Q_3$ représente le radical hydroxyle et $Q_4$ représente le radical caféoylmaloyle de formule (IVa) ou de formule (IVb) ; et
  - Le composé de formule ($Ic_2$) correspondant à la formule (I) pour laquelle $Q_1$ et $Q_4$ représentent le radical caféoyle de formule (II), $Q_3$ représente le radical hydroxyle et $Q_5$ représente le radical caféoylmaloyle de formule (IVa) ou de formule (IVb).

Selon un aspect plus particulier de la présente invention, dans la composition (ES) telle que définie précédemment, le composé de formule (Ia) correspondant à la formule (I) telle que définie précédemment et pour laquelle $Q_1$ représente le radical maloyl de formule (IIIa), $Q_3$ et $Q_4$ et $Q_5$, identiques, représentent le radical caféoyl de formule (II).

Selon un aspect plus particulier de la présente invention, dans la composition (ES) telle que définie précédemment, le composé de formule (Ia) correspondant à la formule (I) telle que définie précédemment et pour laquelle $Q_1$ représente le radical maloyl de formule (IIIb), $Q_3$ et $Q_4$ et $Q_5$, identiques, représentent le radical caféoyl de formule (II).

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVa) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVb) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$, identiques, représentent le radical caféoyl de formule (II) ; $Q_3$ représente le radical -OH ; $Q_4$ représente le radical caféoylmaloyl de formule (IVa).

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$, identiques, représentent le radical caféoyl de formule (II) ; $Q_3$ représente le radical -OH ; $Q_4$ représente le radical caféoylmaloyl de formule (IVb).

Selon un aspect plus particulier de la présente invention, dans la composition (ES) telle que définie précédemment, le composé de formule (Ic) est le composé de formule ($Ic_2$), correspondant à la formule (I) telle que définie précédemment et pour laquelle $Q_1$ et $Q_4$, identiques, représentent le radical caféoyl de formule (II) ; $Q_3$ représente le radical -OH ; $Q_5$ représente le radical caféoylmaloyl de formule (IVa).

Selon un aspect plus particulier de la présente invention, dans la composition (ES) telle que définie précédemment, le composé de formule (Ic) est le composé de formule ($Ic_2$), correspondant à la formule (I) telle que définie précédemment et pour laquelle $Q_1$ et $Q_4$, identiques, représentent le radical caféoyl de formule (II) ; $Q_3$ représente le radical -OH ; $Q_5$ représente le radical caféoylmaloyl de formule (IVb).

Selon un aspect plus particulier de la présente invention, la composition (ES) telle que définie précédemment comprend au moins :

- un composé de formule (Ia) telle que définie précédemment pour laquelle $Q_1$ représente le radical maloyl de formule (IIIa) ou le radical maloyl de formule (IIIb), et
- un composé de formule (Ib) telle que définie précédemment et pour laquelle $Q_1$ représente le radical caféoylmaloyl de formule (IVa) ou le radical caféoylmaloyl de formule (IVb), et
- un composé de formule ($Ic_1$) telle que définie précédemment et pour laquelle $Q_4$ représente le radical caféoylmaloyl de formule (IVa) ou le radical caféoylmaloyl de formule (IVb).

Selon un aspect plus particulier de la présente invention, la composition (ES) telle que définie précédemment comprend au moins :

- un composé de formule (Ia) telle que définie précédemment pour laquelle $Q_1$ représente le radical maloyl de formule (IIIa) ou le radical maloyl de formule (IIIb), et
- un composé de formule (Ib) telle que définie précédemment et pour laquelle $Q_1$ représente le radical caféoylmaloyl de formule (IVa) ou le radical caféoylmaloyl de formule (IVb), et
- un composé de formule ($Ic_2$) telle que définie précédemment et pour laquelle $Q_5$ représente le radical caféoylmaloyl de formule (IVa) ou de formule (IVb).

Selon un aspect particulier de la présente invention, le solvant organique ($SO_1$) présent dans la composition (C1) telle que définie précédemment est choisi parmi les éléments du groupe constitué par le 1,2-propanediol, le 1,3-propanediol, et le 2-méthyl-2,4-pentanediol.

Selon un autre aspect particulier de la présente invention, la composition ($C_1$) comprend pour 100% de sa masse :

- De 60,0% massique à 75,0% massique de 1,2-propanediol,
- De 0,1% massique à 2,0% massique d'une composition (ES) comprenant une quantité massique $x_1$, exprimée en

équivalent massique de l'acide 1-O-(2-caféoyl)maloyl-3,5-O-dicaféoyl quinique, supérieure ou égale à 200 mg/ d'au moins le composé de formule (Ia) tel que défini à la revendication 2, et d'au moins le composé de formule (Ib) tel que défini à la revendication 2, et d'au moins le composé de formule (Ic) tel que défini à la revendication 2

- De 20,0% massique à 35,0% massique d'eau.

La composition (C1) peut être préparée par simple mélange de ses constituants, à température comprise entre 20°C et 60°C, plus particulièrement entre 20°C et 40°C, et encore plus particulièrement entre 20°C et 30°C , et sous agitation mécanique de type ancre à une vitesse comprise entre 50 tours/minute et 150 tours/minute.

L'étape a) de mise en culture en conditions hors sol (ou dite en aéroponie) de la plante *Arctium lappa* se réalise selon les conditions standard connues de l'homme du métier, et plus particulièrement celles concernant l'influence de la teneur en azote (2) (3) (4) (5) (6), et de la teneur en phosphore et en potassium présentes dans le milieu de culture. L'étape a) de mise en culture en conditions hors sol se réalise donc en optimisant le ratio azote/phosphore/potassium présent dans le milieu nutritif, et en optimisant le paramètre d'électroconductivité d'un tel milieu nutritif.

L'étape a) est généralement conduite à une température comprise entre 20°C et 40°C, pendant une durée comprise entre 4 et 10 semaines, de façon à obtenir une biomasse ($BM_1$), en quantité importante notamment au niveau racinaire ; l'étape a) est arrêtée lorsque la croissance de la biomasse ($BM_1$) n'est plus observée.

L'application sur la peau d'un tel agent actif cosmétique représentée par la composition ($C_1$) telle que définie précédemment permet :

- de limiter la formation du biofilm de bactéries opportunistes pathogènes, comme par exemple *Staphylococcus. Aureus,* sans altérer la présence de bactéries commensales telles que *Staphylococcus. Epidermidis,*
- de protéger les sébocytes de la surproduction de lipides, et
- de renforcer une activité anti-lipase de la peau ou du cuir chevelu ;

effets responsables du développement du sébum sur la peau et sur le cuir chevelu, et par conséquent des effets inesthétiques associés.

Il est également décrit un procédé pour empêcher ou ralentir l'apparition des signes inesthétiques liés à la présence de sébum en excès sur la peau et/ou le cuir chevelu, caractérisé en ce qu'il comprend au moins une étape $a_1)_2$) comprenant au moins un excipient cosmétiquement acceptable (E) et une composition ($C_1$) consistant en, pour 100% de sa masse :

a) de 60,0% massique à 75,0% massique de 1,2-propanediol,

b) de 0,1% massique à 2,0% massique d'une composition (ES) comprenant une quantité massique $x_1$, exprimée en équivalent massique de l'acide 1-O-(2-caféoyl)maloyl-3,5-O-dicaféoyl quinique, supérieure ou égale à 200 mg/ d'au moins le composé de formule (Ia) tel que défini ci-dessus, et d'au moins le composé de formule (Ib) tel que défini ci-dessus, et d'au moins le composé de formule (Ic) tel que défini ci-dessus

c) de 20,0% massique à 35,0% massique d'eau.

Selon un aspect particulier le procédé pour empêcher ou ralentir l'apparition des signes inesthétiques liés à la présence de sébum en excès sur la peau et/ou le cuir chevelu, comprend au moins une étape $a_1)_2$) comprenant au moins un excipient cosmétiquement acceptable (E) et une composition ($C_1$) consistant en, pour 100% de sa masse :

a) de 60,0% massique à 75,0% massique de 1,2-propanediol, De 64,2% massique à 75% massique, plus particulièrement de 68% massique à 75% massique, et encore plus particulièrement de 68% massique à 72% de 1,2-propanediol,

b) de 0,1% massique à 2,0% massique , plus particulièrement de 0,8% massique à 2% massique, et encore plus particulièrement de 1% massique à 2% massique, d'une composition (ES) comprenant une quantité massique $x_1$, exprimée en équivalent massique de l'acide 1-O-(2-caféoyl)maloyl-3,5-O-dicaféoyl quinique, supérieure ou égale à 200 mg/g, plus particulièrement supérieure ou égale à 400 mg/g, et encore plus particulièrement supérieure ou égale à 600 mg/g d'au moins le composé de formule (Ia) tel que défini précédemment, et d'au moins le composé de formule (Ib) tel que défini précédemment, et d'au moins le composé de formule (Ic) tel que défini précédemment.

c) De 20,0% massique à 35,0% massique d'eau, plus particulièrement de 23% massique à 31,2% massique et encore plus particulièrement de 27% massique à 31 % massique d'eau.

Par « quantité efficace », on désigne, dans la définition du procédé tel que défini ci-dessus, une quantité telle que l'activité anti-lipase de la peau traitée soit supérieure à 50% par rapport au témoin, et/ou que la production de lipide par les sébocytes de la peau traitée soit inhibée, et/ou que la formation du biofilm de bactéries pathogènes soit diminuée ou ralentie ou inhibée.

L'expression "à usage topique" utilisée dans la définition de la composition ($C_2$) signifie que ladite composition ($C_2$) est mise en œuvre par application sur la peau, qu'il s'agisse d'une application directe ou d'une application indirecte lorsque ladite composition ($C_2$) est imprégnée sur un support destiné à être mis en contact avec la peau (papier, lingette, textile, dispositif transdermique, etc...).

Ladite composition ($C_2$) est généralement étalée sur la surface de la peau à traiter, puis la peau est massée quelques instants.

L'expression « cosmétiquement acceptable » utilisée dans la définition de la composition ($C_2$) signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

La composition ($C_2$) à usage topique se présente généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition ($C_2$) à usage topique peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, la composition ($C_2$) à usage topique comporte également des excipients et ou des principes actifs habituellement mis en œuvre dans le domaine des formulations à usage topique, en particulier cosmétiques, dermocosmétiques, pharmaceutiques ou dermopharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

Comme exemples de tensioactifs moussants et/ou détergents que l'on peut associer à la composition ($C_1$), on peut citer les tensioactifs moussants et/ou détergents anioniques, cationiques, amphotères ou non ioniques.

Parmi les tensioactifs anioniques moussants et/ou détergents que l'on peut associer à la composition ($C_1$), on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alphaoléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents que l'on peut associer à la composition ($C_2$) à usage topique telle que définie précédemment, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut associer à la composition ($C_1$), on peut citer particulièrement les dérivés d'ammoniums quaternaires.

Parmi les tensioactifs non ioniques moussants et/ou détergents que l'on peut associer à la composition ($C_1$), on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à la composition ($C_1$), on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60

hexyl dicarbamate commercialisé sous l'appellation ELFACOS™ GT2125.

Comme exemples d'agents épaississants et/ou gélifiants que l'on peut associer à la composition ($C_1$) à usage topique telle que définie précédemment, on peut citer les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

$$CH_2=C(R'_3)-C(=O)-[CH_2-CH_2-O]n'-R'_4 \qquad (VIII)$$

dans laquelle $R'_3$ représente un atome d'hydrogène ou un radical méthyle, R'4 représente un radical alkyle linéaire ou ramifié comportant de huit à trente atomes de carbone et n' représente un nombre supérieur ou égal à un et inférieur ou égal à cinquante.

Comme exemples d'agents épaississants et/ou gélifiants que l'on peut associer à la composition ($C_1$), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à la composition ($C_1$), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à la composition ($C_1$), on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à la composition ($C_1$), on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à la composition ($C_1$), on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à la composition ($C_1$), on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à la composition ($C_2$) à usage topique telle que définie précédemment, on peut citer les xylènes sulfonates, les cumènes sulfonates, l'hexylpolyglucoside, le 2- éthylhexylpolyglucoside, le n-heptylpolyglucoside.

Comme exemples d'agents tensioactifs émulsionnants que l'on peut associer à la composition ($C_1$), on peut citer les tensioactifs non ioniques, des tensioactifs anioniques, des tensioactifs cationiques.

Comme exemples de tensioactifs non-ioniques émulsionnants que l'on peut associer à la composition ($C_1$), on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters deméthylglucoside ; les alkylpolyglycosides comportant un radical aliphatique, linéaire ou ramifié, saturé ou insaturé, et comportant de 14 à 36 atomes de carbone, comme le tétradécylpolyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkylpolyglycosides tels que décrits précédemment, par exemple les compositions commercialisées sous les noms MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples de tensioactifs anioniques que l'on peut associer à la composition ($C_1$), on peut citer le glycéryl stéarate citrate, le cétéarylsulfate, les savons comme le stéarate de sodium ou le stéarate de triéthanolammonium, les dérivés N-acylés d'acides aminés salifiés, par exemple le stéaroyl glutamate.

Comme exemples de tensioactifs cationiques émulsionnants que l'on peut associer à la composition ($C_1$), on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer à la composition ($C_1$), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer à la composition ($C_1$), on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica

Comme exemples d'agents déodorants que l'on peut associer à la composition ($C_1$), on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à la composition ($C_1$), on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à la composition ($C_1$), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à la composition ($C_1$), on peut citer les vitamines et leurs dérivés, notamment leurs esters, tels que le rétinol (vitamine A) et ses esters (palmitate de rétinyle par exemple),

l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE™MSH, le SEPICALM™VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysats partiels de protéines N-acylés ; les acides aminés ; les peptides ; les hydrolysats totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photovieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginkgo biloba, de lierre, de marron d'inde, de bambou, de ruscus, de petit houx, de *centella asiatica,* de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caroténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI : Butylene glycol , Palmitoyl Tripeptide-40) par la société LIPOTEC, les sucres et les dérivés de sucres par exemple le produit commercialisé sous le nom de

marque Tanositol™ (nom INCI : inositol) par la société Provital, le produit commercialisé sous le nom de marque Thalitan™ (ou Phycosaccharide™ AG) par la société CODIF international (nom INCI : Aqua and Hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP1515688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1 515 688 A2.

Comme exemples d'agents antioxydants que l'on peut associer à la composition ($C_1$), on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE□ GL 47S commercialisé par la société Akzo Nobel sous le nom INCI : Tetrasodium Glutamate Diacetate.

Comme exemples de filtres solaires que l'on peut associer à la composition ($C_1$), on peut citer tous ceux figurant dans la directive cosmétique 76/768/CEE modifiée annexe VII.

Parmi les filtres organiques solaires que l'on peut associer à la composition ($C_2$) à usage topique telle que définie précédemment, on peut citer la famille des dérivés de l'acide benzoïque comme les acides para-aminobenzoïques (PABA), notamment les esters de monoglycérol de PABA, les esters éthyliques de N,N25 propoxy PABA, les esters éthyliques de N,N-diéthoxy PABA, les esters éthyliques de N,Ndiméthyl PABA, les esters méthyliques de N,N-diméthyl PABA, les esters butyliques de N,Ndiméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl- 2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de pméthoxy 2-éthylhexyle), le cinnamate de p-méthoxy 2-éthoxyéthyle, le p-cinnamate de méthoxycyclohexyle, le cinnamate d'éthyl-$\alpha$-cyano-$\beta$-phényle, le cinnamate de 2-éthylhexyl-$\alpha$-cyano-$\beta$-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-5 carboxylate, la 2-hydroxy-4-n-octyloxybenzophénone, la 4-hydroxy-3-carboxybenzophénone ; le 3-(4'-méthylbenzylidène)-d,l-camphre, le 3 (benzylidène)-d,Icamphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl)amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-tbutylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", que l'on peut associer à la composition ($C_1$), on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Il est également décrit une composition ($C_1$) telle que définie précédemment, pour son utilisation dans une méthode de traitement thérapeutique visant à diminuer la quantité de sébum produite par la peau humaine et/ou le cuir chevelu.

Selon un aspect particulier, la composition ($C_1$) telle que définie précédemment est utilisée dans une méthode de traitement thérapeutique visant à diminuer et/ou éliminer et/ou prévenir la formation de comédons (points noirs) et/ou de points blancs (microkystes) et/ou de papules et/ou de pustules et/ou de nodules et/ou de cicatrices, accompagnant des pathologies cutanées et/ou mucosales telles que l'acné.

Par « acné », on entend l'acné vulgaris, l'acné rétentionnelle, l'acné inflammatoire, l'acné kystique, l'acné vulgaire, l'acné papulo-pustuleuse, l'acné conglobata, l'acné du nouveau-né, l'acné des cosmétiques, l'acné excoriée des jeunes filles, l'acné de Majorque, l'acné professionnelle, l'acné médicamenteuse.

La composition ($C_1$) pour son utilisation dans un traitement thérapeutique tel que défini précédemment peut être associée avec des ingrédients actifs pharmaceutiques en particulier dermatologiques.

Bibliographie :

**(1) :** Chan et al., « A review of the pharmacological effects of Arctium lappa », Inflammopharmacol, 2011, 19:245-254).

**(2) :** Pirvu et al.,"Comparative studies on analytical, antioxidant, and antimicrobial activities of a series of vegetal extracts prepared from eight plant species growing in Romania", J planar Chromato 2014.

Les exemples suivants illustrent l'invention, sans toutefois la limiter.

## A) Exemple de préparation

### $A_1$) Exemple de préparation d'une composition ($C_{1A}$) préparée selon l'invention.

Les plantes de Bardane ou *Arctium lappa* ont préalablement été obtenues par germination de graines pendant une durée de 60 jours dans des conditions standard, de façon à atteindre une taille d'environ 10 à 15 centimètres, puis elles sont dépotées pour être placées dans des conditions de culture « hors-sol » ou en milieu aéroponie.

Les racines des plantes de Bardane sont ainsi trempées dans une solution nutritive se caractérisant par une électro-conductivité comprise entre 1,0 et 1,2 millisiemens, et par un ratio massique N/P/K (Azote/Phosphore/Potassium) apportés par l'engrais d'environ 15/10/30. Cette phase de culture en aéroponie est conduite pendant six semaines à une température régulée à 20°C, et permet d'obtenir un rendement racinaire de 754 grammes par mètre carré.

Les racines fraîches de la biomasse ainsi obtenue sont prélevées et immergées pendant 15 minutes dans un bain comprenant un mélange comprenant pour 100% de sa masse, 70% massique de 1,2-propanediol et 30% massique d'eau distillée, à une température de 25°C ; la valeur du pH de l'eau distillée ayant été préalablement réglé à 2,0 $\pm$0,2 par ajout d'une solution à 75% massique d'acide phosphorique. Le ratio de biomasse racinaire ainsi immergées pour un volume de mélange 1,2-propanediol et d'eau préalablement décrit, s'élève à 1,0 kg de biomasse racinaire pour 1 litre de de mélange 1,2-propanediol et d'eau.

A l'issue de l'immersion, les plantes sont sorties de leur bain d'exsudation ($L_1$) qui est conservé et les racines sont égouttées, puis coupées, puis la biomasse restante est remise à macérer pendant une durée de 48 heures dans un bain comprenant un mélange comprenant pour 100% de sa masse, 70% massique de 1,2-propanediol et 30% massique d'eau distillée, à une température de 25°C ; la valeur du pH de l'eau distillée ayant été préalablement réglé à 2,0 $\pm$0,2 par ajout d'une solution à 75% massique d'acide phosphorique. Le ratio de biomasse racinaire ainsi immergées pour un volume de mélange 1,2-propanediol et d'eau préalablement décrit, s'élève à 0,5 kg de biomasse pour 1 litre de de mélange 1,2-propanediol et d'eau.

A l'issue de cette phase de macération, la biomasse est séparée du liquide de macération ($L_2$), ledit liquide ($L_2$) étant par la suite filtré avec un filtre poche de 50 micromètres.

Les liquides ($L_1$) et ($L_2$) sont par la suite regroupés, et une quantité nécessaire de 1,2-propanediol est rajoutée pour ajuster sa teneur massique à 70%, pour obtenir le liquide ($L_3$), qui est par la suite filtré sous membrane de 1 micromètre de façon à le clarifier, et enfin sous filtration stérilisante avec une membrane à 0,2 micromètre, de façon à atteindre la composition ($C_{1A}$).

### $A_2$) Exemple de préparation d'une composition comparative ($C_{comp}$).

Les plantules issues du même lot de graines que celle utilisées pour obtenir les plantules cultivées par la suite en aéroponie (exemple $A_1$), sont utilisées pour une culture en terre desdites plantules pendant une durée de six semaines.

A l'issue de cette période, les plants sont dépotés, les racines fraîches nettoyées, coupées et broyées, puis ledit broyat obtenu est extrait selon un procédé d'extraction liquide-solide conventionnel (macération, agitation, filtration) à l'aide d'un mélange solvant 1,2-proapanediol/eau distillée de 70/30, à une température de 25°C, avec un ratio massique racines fraiches/milieu solvant de 0,5 kg de biomasse pour 1 litre de de mélange 1,2-propanediol et d'eau ; la valeur du pH de l'eau distillée ayant été préalablement réglé à 2,0 $\pm$0,2 par ajout d'une solution à 75% massique d'acide phosphorique.

A l'issue de cette phase d'extraction, la biomasse est séparée du liquide qui est ensuite filtré avec un filtre poche

de 50 micromètres, puis avec une membrane de 1 micromètre de façon à le clarifier, et enfin sous filtration stérilisante avec une membrane à 0,2 micromètre, de façon à atteindre la composition ($C_{comp}$).

**$A_3$) Caractérisation analytique de la composition ($C_{1A}$) préparée selon l'invention et de la composition comparative ($C_{comp}$).**

La composition ($C_{1A}$) préparée selon l'invention et de la composition comparative ($C_{comp}$) ont été caractérisées analytiquement et les caractéristiques sont comprises dans le Tableau 7 ci-dessous.

**Tableau 7**

| Caractéristiques analytiques | Méthode analytique | Composition ($C_{1A}$) préparée selon l'invention | Composition comparative ($C_{comp}$) |
|---|---|---|---|
| Apparence | Visuelle | Liquide jaune | Liquide jaune |
| Teneur en 1,2 propanediol | Chromatographie phase gaz (espace de tête) | 71,4% | 70,0% |
| pH | Selon la norme NFT 73-206 | 3,1 | 3,2 |
| Extrait sec en % massique | Etuve à 105°C, 12 heures | 0,21% | 1,12% |
| Eau en % massique | (Norme NFT 73201) | 28,39% | 28 ,88% |
| Teneur totale en composés de formule (Ia), de formule (Ib), de formule ($Ic_1$) et de formule ($Ic_2$) exprimée en milligramme/-gramme d'extrait sec dû à la plante | UHPLC-MS<br>Appareil : Shimadzu Nexera X2<br>Colonne : Waters Xterra RP C18 ; 250x4,6 mm<br>Phase mobile (avec qradient) :<br>A) Eau + acide formique<br>B) Acétonitrile<br>Détecteur UV (330 nm) | 613,3 mg/g | 169,5 mg/g |

**B) Mise en évidence des propriétés actives des compositions ($C_{1A}$) préparée selon l'invention et ($C_{comp}$) comparative.**

**$B_1$) Mise en évidence de l'effet de la Composition ($C_{1A}$) sur la surproduction de lipides induite en cas de déséquilibre du microbiote**

*B.1.1. Principe de la méthode*

Le déséquilibre du microbiote cutané survient notamment lorsqu'on observe la prolifération de *Propionibacterium acnes.* Dans ce cas également, *Propionibacterium acnes.* induit l'inflammation de la peau, d'une part en induisant la surproduction de lipides par les sébocytes, et d'autre part en produisant une lipase qui transforme les triglycérides en acides gras libres, qui sont irritants et chimiotactiques pour les neutrophiles.

L'effet de la Composition ($C_{1A}$) sur la surproduction de lipides a été étudié sur des sébocytes de la lignée SEBO662AR (lignée Bioalternatives) sensibles à des stimulii tels que la testostérone pour produire des lipides.

Les sébocytes ont tout d'abord été traités pendant 4 heures avec la composition à tester (ou une référence positive). Puis la testostérone a été ajoutée pendant 7 jours au milieu de culture, avec renouvellement des traitements et de la stimulation au bout de 3 jours. La formation de gouttelettes lipidiques a été détectée en imagerie grâce à la sonde fluorescente Bodipy(r) et les résultats ont été normalisés par comptage des noyaux au Hoechst.

*Eléments statistiques :*

Les valeurs sont exprimées en moyennes +/- sem [standard error of the mean ou erreur type de la moyenne = écart-type / racine (nombre de valeurs)].

Pour chaque traitement :

% stimulation = 100 x [moyenne (cellules + traitement) / moyenne (cellules stimulées avec la testostérone)]

% inhibition = 100 x [moyenne (cellules + traitement) - moyenne (cellules stimulées avec la testostérone) / [moyenne (cellules non traitées) - moyenne (cellules stimulées avec la testostérone)]

L'analyse statistique des résultats a été menée à l'aide d'un test de Student bilatéral avec un seuil de significativité fixé à 5%, en comparant les séries de valeurs deux à deux.

On considérera qu'une différence entre l'efficacité de deux produits est :

- Significative si $p < 0,05$ ;
- Dite « à la limite de significativité » si $0,05 \leq p < 0,1$ ;
- Et non significative si $p > 0,1$.

*B.1.2. Résultats :* les résultats obtenus sont consignés dans le tableau 8 ci-dessous. Pour les cellules stimulées avec la testostérone : *** p<0,001 vs cellules non stimulées. Pour cellules stimulées avec la testostérone et traitées avec les produits à l'essai : *** p<0,001; ** p<0,01 vs cellules stimulées.

**Tableau 8**

| | Lipides (intensité de fluorescence) / nombre de cellules | % Stimulation | % Inhibition |
|---|---|---|---|
| Cellules non stimulées | 1610 +/- 190 | 31 | 100 |
| Cellules stimulées avec la testostérone 1 nM sans traitement | 5179 +/- 224*** | 100 | 0 |
| Cellules stimulées avec la testostérone 1 nM + référence positive (dutastéride 1 μM) | 1152 +/- 133*** | 22 | 113 |
| Cellules stimulées avec la testostérone 1 nM + Composition ($C_{1A}$) 0,001% extrait sec dû à la plante | 2361 +/- 151*** | 46 | 79 |
| Cellules stimulées avec la testostérone 1 nM + Composition ($C_{AComp}$) 0,001% extrait sec dû à la plante | 3452 +/- 108**<br>p = 0,006 vs PAT Bardane | 67 | 48 |

Sur ce même modèle, le composé de formule (Ia), tel que décrit précédemment, a été testé à différentes concentrations et les résultats sont consignés dans le tableau 9 ci-dessous.

**Tableau 9**

| | Lipides (intensité de fluorescence) / nombre de cellules | % Inhibition | % Stimulation |
|---|---|---|---|
| Cellules non stimulées (contrôle) | 6482 +/- 1102 | 100 | -90 |
| Cellules stimulées avec la testostérone 1 nM | 62464 +/- 5067*** | 0 | 0 |
| Cellules stimulées avec la testostérone 1 nM + référence positive (dutastéride 1 μM) | 11478 +/- 8126*** | 91 | -82 |
| Cellules stimulées avec la testostérone 1 nM + Composé de formule (Ia) à 0,0012% | 75511 +/- 1856 | -23 | 21 |
| Cellules stimulées avec la testostérone 1 nM + Composé de formule (Ia) à 0,006% | 41222 +/- 2741* | 38 | -34 |
| Cellules stimulées avec la testostérone 1 nM + Composé de formule (Ia) à 0,012% | 44271 +/- 2649* | 32 | -29 |

*B.1.3. Interprétation et conclusions*

Les mesures consignées dans le tableau 8 montrent que le traitement par la composition préparée selon l'invention ($C_{1A}$) des cellules stimulées par la testostérone, permet d'inhiber la production de lipides de 79% par rapport aux cellules stimulées et non traitées, alors que le traitement par la composition comparative ($C_{comp}$) permet d'inhiber la production de lipides de 48% par rapport aux cellules stimulées et non traitées.

De même, les mesures consignées dans le tableau 9 montrent que le traitement par la composition préparée selon l'invention ($C_{1A}$) des cellules stimulées par la testostérone, permet de diminuer la stimulation de la production de lipide de 54% par rapport aux cellules stimulées et non traitées, alors que le traitement par la composition comparative ($C_{comp}$) permet de diminuer la stimulation de la production de lipide de 33% par rapport aux cellules stimulées et non traitées.

Les mesures consignées dans le tableau 9 montrent que le traitement par le composé de formule (Ia), par des teneurs respectives de 0,006% et de 0,012 %, des cellules stimulées par la testostérone, permet d'inhiber la production de lipides respectivement de 38% et de 32% par rapport aux cellules stimulées et non traitées, alors que le traitement par 0,0012% par le composé de formule (Ia) ne permet pas d'inhiber la production de lipides.

La composition préparée selon l'invention ($C_{1A}$), et le composé de formule (Ia) à une teneur 0,006% massique, permettent de limiter la production de lipides par les sébocytes.

**$B_2$) Mise en évidence de l'effet de la composition ($C_{1A}$) sur la production de lipides par évaluation de l'activité anti-lipase**

*B.2.1. Principe de la méthode*

Il s'agit d'étudier la capacité d'une composition à provoquer et réguler l'activité de l'enzyme lipase par une méthode *in tubo,* ladite enzyme ayant une action inflammatoire.

La lipase présente la capacité de transformer le 1,2-diglycéride, incolore, en glycérol qui est d'une couleur rose.

Les échantillons à l'essai d'évaluation sont mis *in tubo* en présence de lipase et de 1,2-diglycéride et l'absorbance des échantillons est mesurée au spectrophotomètre à la longueur d'onde 570 nm dès la fin de leur préparation, puis au bout de 60 minutes d'incubation à 37°C dans les mêmes conditions spectrales. Grâce à une gamme de glycérol, l'activité lipase des échantillons peut être calculée, ainsi que le pourcentage d'inhibition selon les formules suivantes :

Activité lipase = (quantité de glycérol formée entre 0 et 60 minutes) / ( 60 minutes x volume échantillon )

Pourcentage d'inhibition = 100 x [ (activité lipase du groupe témoin) - (activité lipase du produit à l'essai) ] / (activité lipase du groupe témoin)

*Eléments statistiques :*

Les valeurs sont exprimées en moyennes +/- écart-type.

L'analyse statistique des résultats a été menée à l'aide d'un test de Student bilatéral avec un seuil de significativité fixé à 5%, en comparant les séries de valeurs deux à deux.

On considérera qu'une différence entre l'efficacité de deux produits est :

- Significative si $p < 0,05$ ;
- Dite « à la limite de significativité » si $0,05 \leq p < 0,1$ ;
- Et non significative si $p > 0,1$.

*B.2.2. Résultats obtenus*

Les résultats obtenus sont consignés dans le tableau 10 ci-dessous (*** p<0,001 vs témoin).

**Tableau 10**

| Produits testés | Activité lipase (mU/mL) | % d'inhibition |
|---|---|---|
| Témoin | 4,21 +/- 0,02 | 0 |
| Référence positive (Vitamine C) | 2,11 +/- 0,01 | 50*** |

(suite)

| Produits testés | Activité lipase (mU/mL) | % d'inhibition |
|---|---|---|
| | | |
| Composition ($C_{1A}$) à 1% massique | 0,92 +/- 0,01 | 78*** |
| Propylène Glycol 70% à 1% | 6,40 +/- 0,05 | - |

Sur ce même modèle, le composé de formule (Ia), tel que décrit précédemment, a été testé à différentes concentrations et les résultats sont consignés dans le tableau 11 ci-dessous.

**Tableau 11**

| | | Activité lipase (mU/mL) | % d'inhibition de la lipase |
|---|---|---|---|
| Témoin | | 4,72 +/- 0,07 | 0 |
| Référence positive (Vitamine C) | | 1,36 +/- 0,01 | 71 *** |
| Composé de formule (Ia) | 0,0012% massique | 1,02 +/- 0,01 | 78*** |
| | 0,0006% massique | 2,16 +/- 0,07 | 54*** |
| | 0,00024% massique | 4,15 +/- 0,03 | 12** |
| | 0,00012% massique | 4,65 +/- 0,03 | 1 |

*B.2. 3. Analyse des résultats*

Les mesures consignées dans le tableau 10 montrent que le traitement par la composition préparée selon l'invention ($C_{1A}$) réduit de façon très significative l'activité de la lipase, puisque le pourcentage d'inhibition mesuré est de 78%.

De même, les mesures consignées dans le tableau 11 montrent que le traitement par le composé de formule (Ia), par des teneurs respectives de 0,0006% et de 0,0012 %, réduit de façon très significative l'activité de la lipase, puisque les pourcentages d'inhibition mesurés sont respectivement de 54% et 78%.

La composition préparée selon l'invention ($C_{1A}$), et le composé de formule (Ia) à une teneur 0,0006% massique, permettent de limiter la production d'acides gras sur la peau, et par conséquent de diminuer les effets inesthétiques liés à l'acné.

**$B_3$) Conclusions générales sur les évaluations biologiques mettant en œuvre la composition préparée selon l'invention ($C_{1A}$).**

Les évaluations expérimentales de cette section ont démontré que la composition préparée selon l'invention ($C_{1A}$), et le composé de formule (Ia) compris dans ladite composition ($C_{1A}$) à une teneur massique minimale de 0,006%, permettent de limiter la production de lipides par les sébocytes et par l'inhibition de l'activité de la lipase.

Il en résulte que la composition préparée selon l'invention ($C_{1A}$) peut être utilisée dans le but d'empêcher ou de ralentir l'apparition des signes inesthétiques liés à la présence de sébum sur la peau et/ou le cuir chevelu, ou bien de les éliminer, comme par exemple un aspect luisant, un aspect gras, une sensation collante de la peau ou du cuir chevelu, la présence de comédons fermés (points blancs) et les comédons ouverts (points noirs) sur la peau.

**C) Formulations**

Dans les formules suivantes, les pourcentages sont exprimés en poids de la formulation.

**C1) - Emulsion Huile-dans-eau dermo-purifiante**

| Formule | |
|---|---|
| Eau | q.s. 100% |

(suite)

| Formule | |
|---|---|
| Glycérine | 3% |
| Solagum™AX | 0,3% |
| Montanov™202 | 2% |
| Lanol™ 99 | 7% |
| Cétiol™OE | 3% |
| Lanol™P | 0,25% |
| Sepiplus™400 | 0,8% |
| Euxyl™PE9010 | 1% |
| Sensiva™PA40 | 0,5% |
| Composition ($C_{1A}$) | 1% |
| Acide lactique à 20% | qs pH = 5,5 |

**C2) - Emulsion Huile-dans-eau anti-sébum**

| Formule | |
|---|---|
| Eau | qsp 100% |
| Montanov™202 | 3% |
| Montanov™14 | 1,5% |
| Pelemol™BB | 2% |
| Beurre de Karité | 1,5% |
| Phytosqualane | 3% |
| Huile de jojoba | 3% |
| Triglycéride C8-C10 | 3% |
| DUB ISIP | 3% |
| D,L α-tocophérol | 0,1% |
| Solagum™Tara | 0,6% |
| Composition ($C_{1A}$) | 2% |
| Acide sorbique | 0,3% |
| Soude 48% | 0,07% |

**C3- Sérum apaisant**

| Formule | |
|---|---|
| Sepimax™Zen | 0,5% |
| Eau | qsp 100% |
| Butylène glycol | 2% |
| Aquaxyl™ | 2% |
| Composition ($C_{1A}$) | 1% |
| Montanox 20 | 1% |
| Phenoxyethanol & Ethylhexyl Glycerin | 0,80% |

SOLAGUM™AX est un mélange de gomme d'acacia et de gomme de xanthane utilisé comme agent émulsionnant et commercialisé par la société SEPPIC ;
MONTANOV™202 (nom INCI : Arachidyl Alcohol & Behenyl Alcohol & Arachidyl Glucoside) est un agent émulsionnant commercialisé par la société SEPPIC ;

LANOL™ 99 est de l'isononanoate d'isononyle commercialisé par la société SEPPIC ;
Cétiol™OE (nom INCI : Dicaprylyl ether) est une phase grasse commercialisée par la société BASF ;
LANOL™ P est du glycol palmitate commercialisé par la société SEPPIC ;
Sepiplus™400 (nom INCI : Polyacrylate-13 & Polyisobutene & Polysorbate 20) est un agent épaississant polymérique commercialisé par la société SEPPIC ;
Euxyl™PE9010 (nom INCI : phenoxyethanol and ethylhexylglycerin) est un agent conservateur commercialisé par la société Schülke & Mayr
Sensiva™PA40 (nom INCI : Phenethyl Alcohol (and) Ethylhexylglycerin) est un agent anti-microbien commercialisé par la société Schülke & Mayr
Montanov™14 (nom INCI : Myristyl Alcohol & Myristyl Glucoside) est un agent émulsionnant commercialisé par la société SEPPIC ;
Pelemol™BB est du Behenyl Behenate commercialisé par la société PHOENIX Chemical ; Solagum™Tara est une gomme de Tara utilisée comme agent émulsionnant et commercialisé par la société SEPPIC ;
SEPIMAX™Zen (nom INCI : polyacrylate crosspolymer-6) est un agent épaississant, émulsionnant et stabilisant
AQUAXYL™ (nom INCI : Xylitylglucoside and Anhydroxylitol and Xylitol) : Composition hydratante commercialisée par la société SEPPIC ;
Montanox™ 20 (nom INCI : Polysorbate 20) est un agent émulsionnant de type huile-dans-eau commercialisé par la société SEPPIC.

## Revendications

**1.** Procédé de préparation d'une composition (C1) comprenant pour 100% de sa masse :

**a)** - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;
**b)** - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

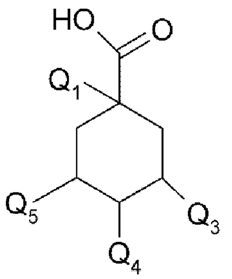

(I),

dans laquelle $Q_1$, $Q_3$, $Q_4$ et $Q_5$ représentent indépendamment les uns des autres, le radical hydroxyle ou un ses sels ou un radical choisi parmi :

**(i)** - Le radical caféoyle de formule (II) :

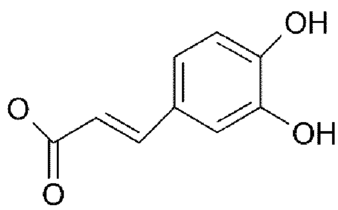

(II)

**(ii)** - Le radical maloyle de formule (IIIa) ou (IIIb) :

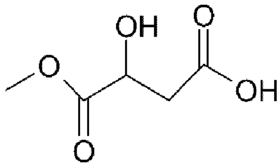 (IIIa) 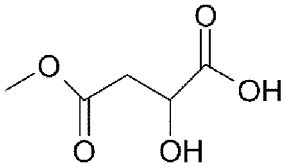 (IIIb) ;

**(iii)** - Le radical caféoyl maloyle de formule (IVa) ou (IVb) :

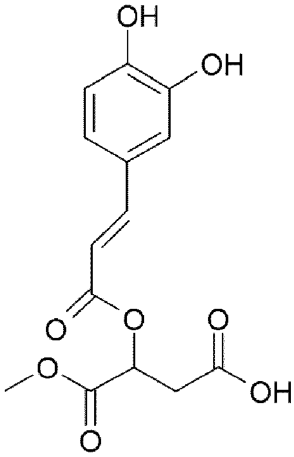 (IVa) 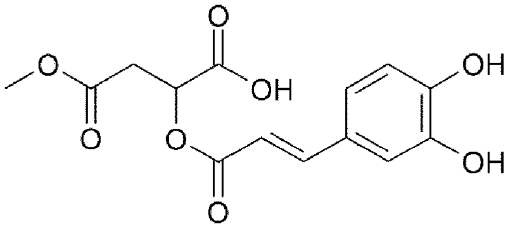 (IVb)

**(iv)** - Le radical maloyl caféoyle de formule (Va), (Vb), (Vc) ou (Vd),

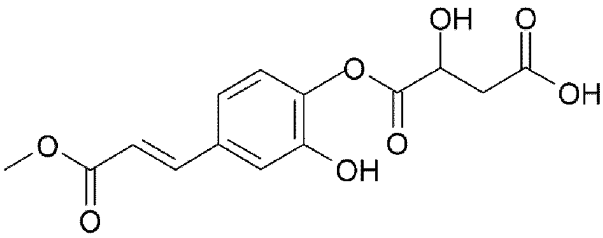

(Va)

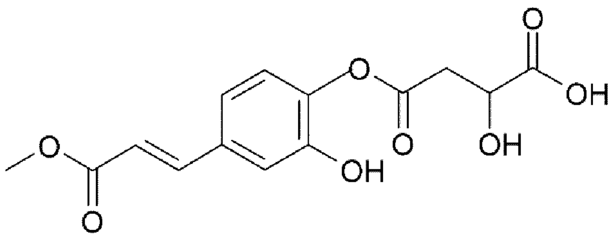

(Vb)

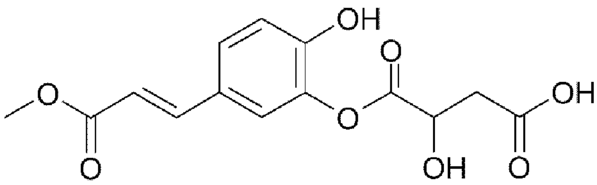 (Vc),

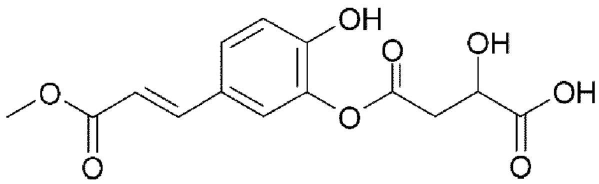

(Vd)

étant entendu qu'au moins un de ces radicaux $Q_1$, $Q_3$, $Q_4$ et $Q_5$ ne représente ni le radical - OH ; ni un de ses sels ; et

- c) - De 20,0% massique à 35,0% massique d'eau,

pour empêcher ou ralentir l'apparition des signes inesthétiques liés à la présence de sébum en excès sur la peau et/ou le cuir chevelu chez l'être humain ;
ledit procédé comprenant les étapes suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/milieu ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;
- Une étape c) de séparation des racines de la biomasse à l'issue du traitement défini à l'étape b), pour isoler une phase liquide ($L_1$) ;
- Une étape d) d'immersion de ladite biomasse ($BM_2$) issue de l'étape c) dans ledit milieu ($S_1$); dans un ratio biomasse ($BM_2$)/milieu ($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$) ,
- Une étape f) de filtration de ladite phase liquide ($L_2$) obtenue à l'étape e), pour isoler une phase liquide liquide ($L_3$),
- Une étape g) de mélange des dites phases liquides ($L_1$) et ($L_3$), puis si nécessaire d'addition d'eau et/ou dudit solvant organique ($SO_1$), de façon à obtenir la composition ($C_1$).

**2.** Procédé selon la revendication 1, **caractérisée en ce que** ladite composition (ES) comprend :

- Au moins un composé de formule (Ia) correspondant à la formule (I) pour laquelle $Q_1$ représente le radical maloyle de formule (IIIa) ou de formule (IIIb) et $Q_3$ et $Q_4$ et $Q_5$ identiques, représentent chacun le radical caféoyle de formule (II) ;
- Un composé de formule (Ib) correspondant à la formule (I) pour laquelle $Q_1$ représente le radical caféoylmaloyle de formule (IVa) ou de formule (IVb), $Q_3$ et $Q_5$ représentent chacun le radical caféoyle de formule (II) et $Q_4$ représente le radical hydroxyle, et
- Au moins un composé de formule (Ic) choisi parmi :

  - Le composé de formule ($Ic_1$) correspondant à la formule (I) pour laquelle $Q_1$ et $Q_5$ représentent chacun le radical caféoyle de formule (II), $Q_3$ représente le radical hydroxyle et $Q_4$ représente le radical caféoylmaloyle de formule (IVa) ou de formule (IVb) ; et
  - Le composé de formule ($Ic_2$) correspondant à la formule (I) pour laquelle $Q_1$ et $Q_4$ représentent le radical caféoyle de formule (II), $Q_3$ représente le radical hydroxyle et $Q_5$ représente le radical caféoylmaloyle de formule (IVa) ou de formule (IVb).

**3.** Procédé selon la revendication 2, pour laquelle la composition ($C_1$) comprend pour 100% de sa masse :

- De 60,0% massique à 75,0% massique de 1,2-propanediol,
- De 0,1% massique à 2,0% massique d'une composition (ES) comprenant une quantité massique $x_1$, exprimée

en équivalent massique de l'acide 1-O-(2-caféoyl)maloyl-3,5-O-dicaféoyl quinique, supérieure ou égale à 200 mg/g d'au moins le composé de formule (Ia), et d'au moins le composé de formule (Ib), et d'au moins le composé de formule (Ic)

- De 20,0% massique à 35,0% massique d'eau.

## Patentansprüche

**1.** Verfahren zur Herstellung einer Zusammensetzung (C1), umfassend, bezogen auf 100% ihrer Masse:

a) - 60,0 Gew.-% bis 75,0 Gew.-% eines organischen Lösungsmittels ($SO_1$), ausgewählt aus 1,2-Propandiol, 1,3-Propandiol, 1,4-Butandiol, 1,3-Butandiol, 1,2-Butandiol, 2-Methyl-2,4-pentandiol, 1,6-Hexandiol, 1,8-Octandiol oder einer Mischung dieser Verbindungen;

b) - 0,1 Gew.-% bis 2,0 Gew.-% einer Zusammensetzung (ES), umfassend eine Massenmenge x1, ausgedrückt in Massenäquivalent der 1-O-(2-caffeoyl)maloyl-3,5-O-dicaffeoylchinasäure, größer oder gleich 200 mg/g mindestens einer Verbindung der allgemeinen Formel (I):

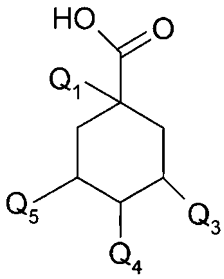

(I)

in der $Q_1$, $Q_3$, $Q_4$ und $Q_5$ unabhängig voneinander das Hydroxylradikal oder eines seiner Salze oder ein Radikal darstellen, das ausgewählt ist aus:

(i) - Dem Caffeoylradikal der Formel (II):

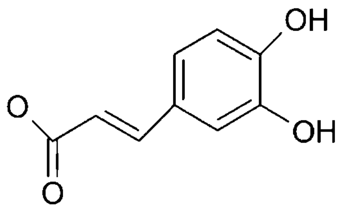

(II)

(ii) - Dem Maloylradikal der Formel (IIIa) oder (IIIb):

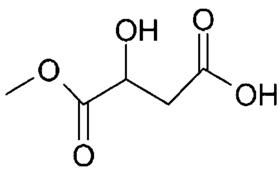

(IIIa)

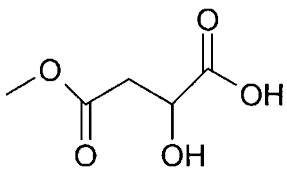

(IIIb) ;

(iii) - Dem Caffeoylmaloylradikal der Formel (IVa) oder (IVb):

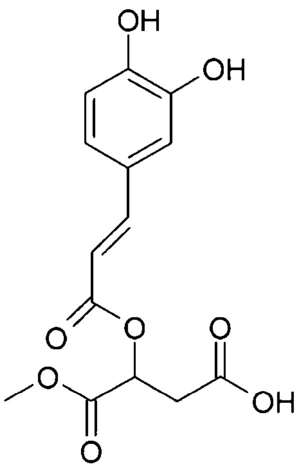

(IVa)

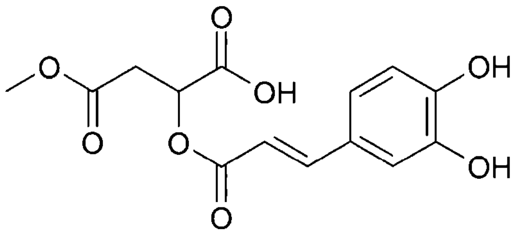

(IVb)

(iv) - Dem Maloylcaffeoylradikal der Formel (Va), (Vb), (Vc) oder (Vd),

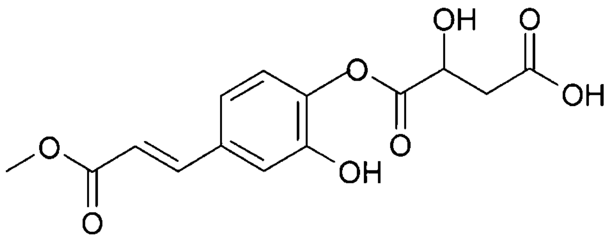

(Va)

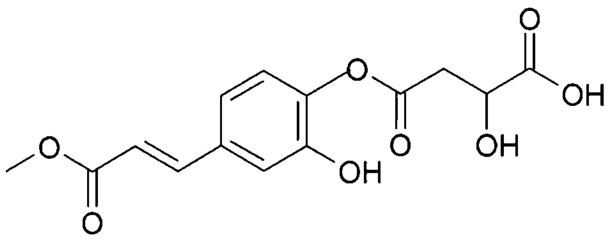

(Vb)

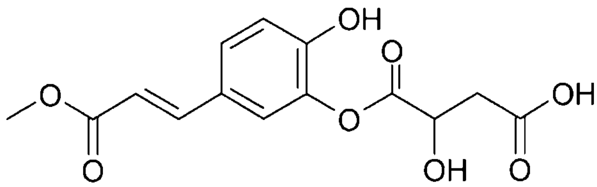

(Vc),

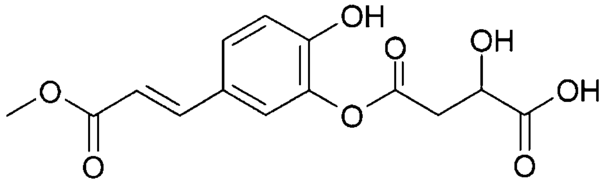

(Vd)

wobei davon ausgegangen wird, dass mindestens eines dieser Radikale $Q_1$, $Q_3$, $Q_4$ und $Q_5$ weder das Radikal -OH; noch eines seiner Salze darstellt; und

c) - 20,0 Gew.-% bis 35,0 Gew.-% Wasser, um das Auftreten von unästhetischen Anzeichen im Zusammenhang mit der Anwesenheit von überschüssigem Talg auf der Haut und/oder der Kopfhaut beim Menschen zu verhindern oder zu verlangsamen;

wobei das Verfahren die folgenden Schritte umfasst:

- Ein Schritt a) der Kultivierung der Pflanze Arctium lappa unter erdlosen Bedingungen, die mit einer Nährlösung versorgt wird, um eine Biomasse ($BM_1$) zu erhalten;
- Ein Schritt b) des Eintauchens der Wurzeln der genannten Biomasse ($BM_1$), die in Schritt a) zuvor erhalten wurde, in ein Medium ($S_1$), so dass das Verhältnis Biomasse ($BM_1$)/Medium ($S_1$) zwischen 0,5 kg/L und 1,5 kg/L liegt, wobei das genannte Medium ($S_1$) zu 100% seiner Eigenmasse 20 Gew.-% bis 35 Gew.-% Wasser, dessen pH-Wert durch Zugabe einer protischen Säure, ausgewählt aus Schwefelsäure, Phosphorsäure und Salzsäure, auf einen Wert zwischen 1,5 und 3,5 eingestellt wurde, und 65 Gew.-% bis 80 Gew.-% eines organischen Lösungsmittels ($SO_1$) umfasst, das ausgewählt ist aus 1,2-Propandiol, 1,3-Propandiol, 1,4-Butandiol, 1,3-Butandiol, 1,2-Butandiol, 2-Methyl-2,4-pentandiol, 1,6-Hexandiol, 1,8-Octandiol oder einer Mischung dieser Diole;
- Ein Schritt c) der Trennung der Wurzeln der Biomasse nach Abschluss der in Schritt b) definierten Behandlung, um eine flüssige Phase ($L_1$) zu isolieren;
- Ein Schritt d) des Eintauchens der genannten Biomasse ($BM_2$) aus Schritt c) in das genannte Medium (S1); in einem Verhältnis Biomasse ($BM_2$)/Medium ($S_1$) zwischen 0,1 kg/L und 1,5 kg/L;
- Ein Schritt e) der Trennung der genannten Biomasse ($BM_2$) nach Abschluss der in Schritt d) definierten Behandlung, um eine flüssige Phase ($L_2$) zu isolieren,
- Ein Schritt f) der Filtration der genannten flüssigen Phase ($L_2$), die in Schritt e) erhalten wurde, um eine flüssige flüssige Phase ($L_3$) zu isolieren,
- Ein Schritt g) des Mischens der genannten flüssigen Phasen ($L_1$) und ($L_3$) und dann, falls erforderlich, der Zugabe von Wasser und/oder des genannten organischen Lösungsmittels ($SO_1$), um die Zusammensetzung ($C_1$) zu erhalten.

**2.** Verfahren nach Anspruch 1, **dadurch gekennzeichnet, dass** die genannte Zusammensetzung (ES) umfasst:

- Mindestens eine Verbindung der Formel (Ia), die der Formel (I) entspricht, für die $Q_1$ das Maloylradikal der Formel (IIIa) oder der Formel (IIIb) darstellt und $Q_3$ und $Q_4$ und $Q_5$, die identisch sind, jeweils das Caffeoylradikal der Formel (II) darstellen;
- Eine Verbindung der Formel (Ib), die der Formel (I) entspricht, für die $Q_1$ das Caffeoylmaloylradikal der Formel (IVa) oder der Formel (IVb) darstellt, $Q_3$ und $Q_5$ jeweils das Caffeoylradikal der Formel (II) darstellen und $Q_4$ das Hydroxylradikal darstellt, und
- Mindestens eine Verbindung der Formel (Ic), ausgewählt aus:

  - Der Verbindung der Formel ($Ic_1$), die der Formel (I) entspricht, für die $Q_1$ und $Q_5$ jeweils das Caffeoylradikal der Formel (II) darstellen, $Q_3$ das Hydroxylradikal darstellt und Q4 das Caffeoylmaloylradikal der Formel (IVa) oder der Formel (IVb) darstellt; und
  - Der Verbindung der Formel ($Ic_2$), die der Formel (I) entspricht, für die $Q_1$ und $Q_4$ jeweils das Caffeoylradikal der Formel (II) darstellen, $Q_3$ das Hydroxylradikal darstellt und Q5 das Caffeoylmaloylradikal der Formel (IVa) oder der Formel (IVb) darstellt.

**3.** Verfahren nach Anspruch 2, wobei die Zusammensetzung ($C_1$) bezogen auf 100% ihrer Masse umfasst:

- 60,0 Gew.-% bis 75,0 Gew.-% 1,2-Propandiol, - 0,1 Gew.-% bis 2,0 Gew.-% einer Zusammensetzung (ES), umfassend eine Massenmenge $x_1$, ausgedrückt in Massenäquivalent der 1-O-(2-caffeoyl)maloyl-3,5-O-dicaffeoylchinasäure, größer oder gleich 200 mg/g mindestens des Compounds der Formel (Ia), und mindestens des Compounds der Formel (Ib), und mindestens des Compounds der Formel (Ic),
- 20,0 Gew.-% bis 35,0 Gew.-% Wasser.

## Claims

**1.** A process for preparing a composition (C1) comprising, for 100% of its mass:

a) - 60.0% by weight to 75.0% by weight of an organic solvent (SO1) chosen from 1,2-propanediol, 1,3-propanediol, 1,4-butanediol, 1,3-butanediol, 1,2-butanediol, 2-methyl-2,4-pentanediol, 1,6-hexanediol, 1,8-octanediol, or a mixture of these compounds;
b) - 0.1% by weight to 2.0% by weight of a composition (ES) comprising a quantity by weight x1, expressed in

weight equivalent of 1-O-(2-caffeoyl) maloyl-3,5-O-dicaffeoyl quinic acid, greater than or equal to 200 mg/g of at least one compound of general formula (I):

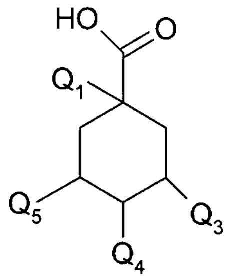

(I)

wherein $Q_1$, $Q_3$, $Q_4$ and $_{Q5}$ independently represent a hydroxyl radical or one of its salts or a radical chosen from:

(i) - The caffeoyl radical of formula (II):

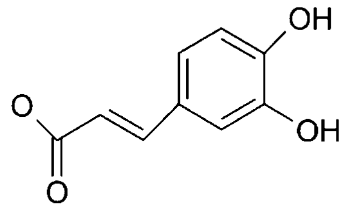

(II)

(ii) - The maloyl radical of formula (IIIa) or (IIIb):

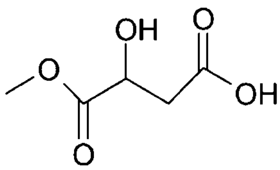

(IIIa)

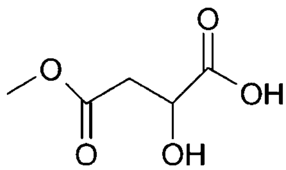

(IIIb) ;

(iii) - The caffeoyl maloyl radical of formula (IVa) or (IVb):

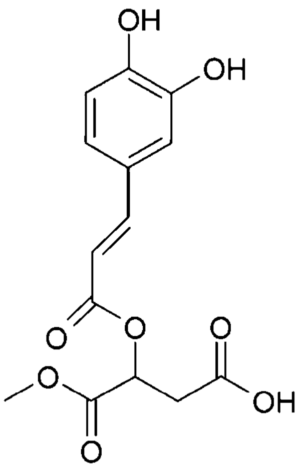

(IVa)

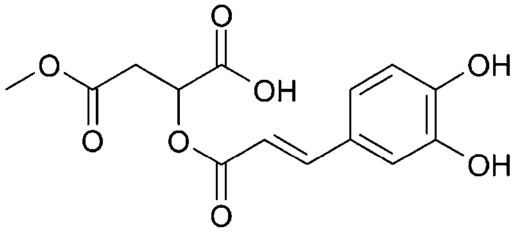

(IVb)

(iv) - The maloyl caffeoyl radical of formula (Va), (Vb), (Vc) or (Vd),

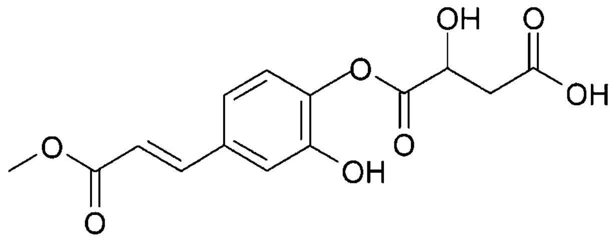

(Va)

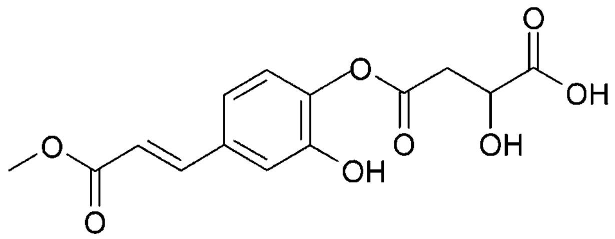

(Vb)

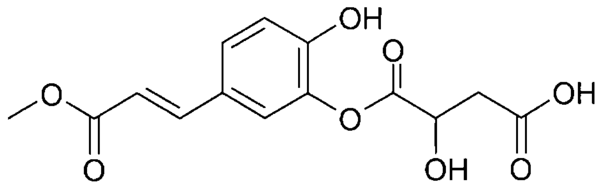

(Vc),

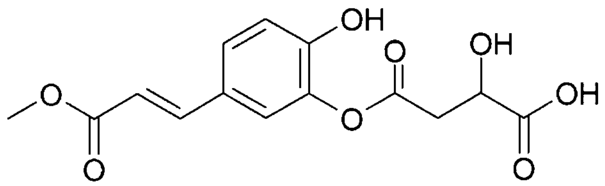

(Vd)

it being understood that at least one of these radicals $Q_1$, $Q_3$, $Q_4$ and $Q_5$ represents neither the radical - OH; nor one of its salts; and

c) - 20.0% by weight to 35.0% by weight of water, for preventing or slowing down the appearance of unaesthetic signs linked to the presence of excess sebum on the skin and/or the scalp in the human being;

said process comprising the following steps:

- Step a) of culturing the plant Arctium lappa under soilless conditions fed by a nutritive solution, so as to obtain a biomass ($BM_1$);
- Step b) of immersing the roots of said biomass ($BM_1$) obtained in step a) above in a medium ($S_1$), such that the biomass ($BM_1$)/medium ($S_1$) ratio is comprised between 0.5 kg/L and 1.5 kg/L, said medium ($S_1$) comprising for 100% of its own mass, 20% to 35% by weight of water whose pH has been adjusted to a value comprised between 1.5 and 3.5 by adding a protic acid chosen from sulfuric acid, phosphoric acid and hydrochloric acid, and 65% to 80% by weight of an organic solvent ($SO_1$) chosen from 1,2-propanediol, 1,3-propanediol, 1,4-butanediol, 1,3-butanediol, 1,2-butanediol, 2-methyl-2,4-pentanediol, 1,6-hexanediol, 1,8-octanediol or a mixture of these diols;
- Step c) of separating the roots of the biomass upon completion of the treatment defined in step b), in order to isolate a liquid phase ($L_1$);
- Step d) of immersing said biomass ($BM_2$) resulting from step c) in said medium ($S_1$); in a biomass ($BM_2$)/medium ($S_1$) ratio comprised between 0.1 kg/L and 1.5 kg/L;
- Step e) of separating said biomass ($BM_2$) upon completion of the treatment defined in step d), in order to isolate a liquid phase ($L_2$),
- Step f) of filtering said liquid phase ($L_2$) obtained in step e), in order to isolate a liquid liquid phase ($L_3$),
- Step g) of mixing said liquid phases ($L_1$) and ($L_3$), then, if necessary, adding water and/or said organic solvent

($SO_1$), so as to obtain the composition ($C_1$).

**2.** The process according to claim 1, **characterized in that** said composition (ES) comprises:

- At least one compound of formula (Ia) corresponding to formula (I) for which $Q_1$ represents the maloyl radical of formula (IIIa) or of formula (IIIb) and $Q_3$ and $Q_4$ and $Q_5$, being identical, each represent the caffeoyl radical of formula (II);
- A compound of formula (Ib) corresponding to formula (I) for which $Q_1$ represents the caffeoylmaloyl radical of formula (IVa) or of formula (IVb), $Q_3$ and $Q_5$ each represent the caffeoyl radical of formula (II) and $Q_4$ represents the hydroxyl radical, and
- At least one compound of formula (Ic) chosen from:

  - The compound of formula ($Ic_1$) corresponding to formula (I) for which $Q_1$ and $Q_5$ each represent the caffeoyl radical of formula (II), $Q_3$ represents the hydroxyl radical and $Q_4$ represents the caffeoylmaloyl radical of formula (IVa) or of formula (IVb); and
  - The compound of formula ($Ic_2$) corresponding to formula (I) for which $Q_1$ and $Q_4$ each represent the caffeoyl radical of formula (II), $Q_3$ represents the hydroxyl radical and $Q_5$ represents the caffeoylmaloyl radical of formula (IVa) or of formula (IVb).

**3.** The process according to claim 2, wherein the composition ($C_1$) comprises for 100% of its mass:

- 60.0% by weight to 75.0% by weight of 1,2-propanediol, - 0.1% by weight to 2.0% by weight of a composition (ES) comprising a quantity by weight $x_1$, expressed in weight equivalent of 1-O-(2-caffeoyl) maloyl-3,5-0-dicaffeoyl quinic acid, greater than or equal to 200 mg/g of at least the compound of formula (Ia), and of at least the compound of formula (Ib), and of at least the compound of formula (Ic)
- 20.0% by weight to 35.0% by weight of water.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CN 106074663 A
- US 20170136077 A
- US 2017239159 A1
- WO 9600719 A
- EP 0971683 A
- EP 1515688 A2


### Littérature non-brevet citée dans la description


- Human Microbiome Project. National Institute of Health, 2007
- **CHAN et al.** A review of the pharmacological effects of Arctium lappa. *Inflammopharmacol*, 2011, vol. 19, 245-254
- **PIRVU et al.** Comparative studies on analytical, antioxidant, and antimicrobial activities of a series of vegetal extracts prepared from eight plant species growing in Romania. *J planar Chromato*, 2014